(19) Europäisches Patentamt

European Patent Office

Office européen des brevets

(11) **EP 0 746 270 B1**

(12) **EUROPEAN PATENT SPECIFICATION**

(45) Date of publication and mention
of the grant of the patent:
**22.11.2000 Bulletin 2000/47**

(51) Int. Cl.[7]: **A61F 2/14**

(21) Application number: **93902750.4**

(86) International application number:
**PCT/US92/11203**

(22) Date of filing: **24.12.1992**

(87) International publication number:
**WO 94/14390 (07.07.1994 Gazette 1994/15)**

(54) **ORBITAL IMPLANT HAVING SYNTHETIC AND ABSORBABLE COATING**

IMPLANTAT FÜR DIE AUGENHÖHLE MIT RESORBIERBARER BESCHICHTUNG

IMPLANT ORBITAIRE AYANT UN COUCHAGE SYNTHETHIQUE ABSORBABLE

(84) Designated Contracting States:
**AT BE CH DE DK ES FR GB GR IE IT LI LU MC NL PT SE**

(43) Date of publication of application:
**11.12.1996 Bulletin 1996/50**

(73) Proprietor:
**Perry Family Limited Partnership
Rancho Santa Fe, California 92067 (US)**

(72) Inventor: **PERRY, Arthur, C.
Rancho Santa Fe, CA 92067 (US)**

(74) Representative:
**Viering, Jentschura & Partner
Postfach 22 14 43
80504 München (DE)**

(56) References cited:
**EP-A- 0 263 012        SU-A- 633 525
US-A- 4 619 655        US-A- 4 713 076
US-A- 4 904 259        US-A- 4 976 731
US-A- 5 002 583        US-A- 5 007 930
US-A- 5 041 138        US-A- 5 073 114**

## Description

Field of the Invention

This invention relates to implants for the orbital socket of the eye.

Background

Enucleation or evisceration of the eye is performed because of disease or trauma that make the removal of the eye, or the intraocular contents of the eye, necessary. Following such a procedure, the patient normally desires use of an artificial eye to restore a more normal appearance. To satisfactorily fit an artificial eye into the orbital socket, an orbital (herein also called an "ocular") implant must be placed within the orbit to replace the volume that was lost when the eye or its contents was removed. However, the use of an orbital implant and the subsequent fitting of the artificial eye confer more than a cosmetic benefit. They help maintain the normal structure of the eyelids and eyebrows; they aid in normal tear drainage; and, when used in children, they help stimulate normal growth of the orbital bones.

Even though an artificial eye can be made today which has a very realistic appearance it fails to track in conjunction with the normal eye because there is no coupling between the artificial eye and the orbital implant. The artificial eye drifts within the socket and does not track with the normal eye. This lack of tracking is quite apparent and disconcerting to even a casual observer, creating a sense of self-consciousness on the part of the patient. Because of this shortcoming of traditional implants, efforts have been made to attach the eye muscles to the implant and then to attach the artificial eye to the implant. This provided adequate tracking of the artificial eye. However the success was short-lived because, in a brief period of time, the implant is extruded from the orbit. This extrusion of the implant occurred because the fixing of the artificial eye to the implant material exposes the implant to the outside environment. This permitted bacteria to enter, and the implant became chronically infected. This exposure was considered necessary, however, to produce the attachment between the implant and the artificial eye.

A porous integrated orbital implant overcomes these problems. One type of integrated orbital implant is described in U.S. Patent No. 4,976,731. In that publication, the use and preparation of an orbital implant comprising hydroxyapatite is described. The use of porous hydroxyapatite allows integration of the implant with fibrovascular tissue. These porous hydroxyapatite implants, or "PHA" implants, are described in U.S. Patent No. 4,976,731, as providing advantages over other implant materials particularly because such integration of the patient's own tissue allows coupling of the implant to the artificial eye, as well as increased long-term stability of both the artificial eye and the implant.

An implant consisting of a nucleus made from biocompatible material wherein said nucleus is wrapped into a coating of synthetic biocompatible materials such as teflon is described in EP-A-0 263 012. In addition, US-A-4,976,731 discloses an orbital implant comprising a monolytic sphere of hydroxyapatite which can sewn within a scleral sac, preserved dura or other homologous or autologous collagen graft material. Further implants consisting of a rigid core which is coated with a well adhering matrix, in which there are embedded collagen fibers, part of which protrude from the surface of said matrix, are described in US-5,002,583.

A wide variety of other materials have been used for orbital implants, such as ivory spheres, gold globes, silk, catgut, acrylic plastics or silicones, human bone (C.C. Sood et al., International Surgery, Vol. 54, No. 1, p. 1 (1970)), and antigen-free cancellous calf bone, so called "Kiel Bone," (A.C.B. Molteno, et al., Brit. J. Ophthal., Vol. 57, p. 615 (1973) and A.C.B. Molteno, Trans: of the Ophthal. Soc. New Zealand, Vol. 32, p. 36 (1980). These other materials, however, do not provide for significant integration of tissue and vascularization of the implant itself. As described above, and in U.S. Patent No. 4,976,731, these materials are thus disadvantageous in that, when the surrounding tissue heals, the patient risks chronic infection as a result of subsequent procedures necessary to connect the implant to the artificial eye to provide tracking of the artificial eye. Also, the weight of the artificial eye is not supported by the implant. This lack of support puts pressure on the lower lid causing lower lid sagging.

In the past, however, unevenly textured surfaces of orbital implants have required coating so that the surface is smooth and slippery for facile insertion of the orbital implant into the eye socket. Uneven or roughly textured surfaces, such as the surface of a hydroxyapatite orbital implant, may cause trauma to or otherwise tear tissue surrounding the eye socket where the implant is to be inserted. An uneven or rough textured surface does not allow the implant to be placed deeply into the orbital socket. This problem has been addressed by using homologous or autologous materials, such as human or animal sclera, fascia, or dura for a coating. See, U.S. Patent No. 4,976,731 which describes, for example, the use of the patient's own scleral sac in the case of an eviscerated eye (in which all the inner contents of the eye have been removed). If the eyeball has been enucleated (removal of the entire eyeball after severing it from the eye muscles and the optic nerve), other material must be used for coating the orbital implant. Materials such as scleral sacs obtained from cadavers or from eye banks, collagen tissue obtained from tissue banks or from animals, or autologous tissue, from another area of the patient's own body have been used.

These materials may be difficult to obtain or use. For example, scleral sacs obtained from eye banks may be unavailable in locations not in proximity to an eye bank. Moreover, these materials may facilitate the transmission of human or animal disease. Human tissues, in particular, may be capable of transmission of such disease as hepatitis or Acquired Immune Deficiency Syndrome (AIDS). Moreover, some of these materials are perishable, and thus must be transported rapidly and used rapidly. If autologous tissue is used, there is, of course, a certain amount of trauma to the patient himself because another invasive operation is required. Furthermore, typically with the use of human or animal tissue for coating orbital implants, the surgeon must perform such wrapping immediately prior to the implantation, thus, the time of surgery is lengthened and the time the patient must be under anesthesia is prolonged. Also, large-scale production of pre-coated orbital implants is impracticable and unavailable.

As a result of the shortcomings of prior coating materials for orbital implants, there exists an unfilled and long felt need for an orbital implant coating material which is not capable of disease transmission, which causes no detriment or undue trauma to the recipient of the orbital implant, which can be stored and used later without significant problems of perishability, which can be practicably produced in conjunction with the orbital implant itself, which does not lengthen the operation or unduly prolong the time the patient is anesthetized, and which provides a smooth, even surface for the orbital implant.

Relatedly, there also exists a need for means to improve the vascularization of integrated orbital implants. As set forth above, integrated orbital implants, for example, those comprised of hydroxyapatite, allow vascularization of the implant itself. In a certain percentage of the patients (about three to five percent), however, vascularization is impeded or inhibited for no known reason. In addition, an increased rate or degree of vascular integration into the orbital implant may benefit patients by allowing a faster fitting of the improved prosthetic.

The advantages to improved vascularization, either in terms of rate of penetration, or the density of blood vessels formed per unit volume, are clear means to attach the orbital implant to the artificial eye (and thereby provide means for smooth movement of the artificial eye) may occur sooner; and, the strength of the coupling of the orbital implant to the surrounding muscle and scar tissue may be enhanced. With increased vascularization, implant migration and extrusion is decreased. Integration of the tissue is extremely important to properly hold the implant in place.

Therefore, there also exists a need for improved means for vascularization of integrated orbital implants. This improved means for vascularization may be associated with the orbital implant coating, or associated with the orbital implant itself.

Summary of the Invention

The present invention provides of orbital implants by providing synthetic absorbable coating materials. Also, means for coating integrated orbital implants so that the surface is smooth is herein provided. Relatedly, means for improved vascularization of integrated orbital implants are provided. Compositions and methods are described herein.

As used herein, the orbital implants is synonymous with ocular implants. The term "integrated" is used herein to denote those implants into which the orbital tissue of the recipient penetrates. Also as used herein, the term "coat" or "coating" denotes material which is used to cover the implant. One type of "coating" is a non-liquid form of material, such as a sheet-like bio-polymer (described in further detail herein), which is here also described as something which is "wrapped" or a "wrapping."

Synthetic coating materials are provided which virtually eliminate the risk of disease transmission previously resulting from use of human or animal tissues. Also, synthetic coating material provides for a decrease in preparation time in that the orbital implant may be produced without the need for a surgeon to coat the orbital implant immediately prior to insertion into a patient. This would also reduce operative time and time under anesthesia. In addition, the use of synthetic materials eliminates morbidity caused by use of the patient's own tissue, as well as the problems caused by the perishable nature of naturally occurring products. There is thus provided a means for a uniform surface which enables the orbital implant, whether integrated or non-integrated, to freely slide into the orbital socket with minimal damage to the surrounding tissue. As an additional benefit, the use of synthetic materials may improve the rate or robustness of vascularization of integrated orbital implants. One example provided herein is coating a hydroxyapatite integrated orbital implant with plaster of Paris. Other examples include biopolymers. Again, these materials may be suitable for dipping the implant, or may be in solid (or semi-solid) form and may be suitable for wrapping the implant. This also satisfies the need for an implant which may be deeply inserted into the orbital socket, yet permits fibrovascular integration.

Relatedly, provided are means and compositions for improved vascularization of the orbital implant. This improved vascularization involves the addition of vascularizing agents to the orbital implant, or to the coating materials. These agents promote the vascularization of the orbital implant via enhancing the vascularization process from the surrounding tissue. Other advantages to using vascularizing agents may include improved wound healing or other secondary therapeutic benefits to the patient. There is thus provided the inclusion of vascularizing agents either in association with the orbital implant, in association with the coating material, or via the addition of exogenous vascularizing agents at the time of the insertion of the orbital implant, or as an ongoing therapy until the orbital implant is sufficiently integrated.

It is an object of the present invention to provide orbital implants with said synthetic coating material which may be absorbed by the recipient's tissue, yet present extremely low levels, or essentially no risk, of transmission of human disease, which cause no trauma to the patient, which are not unduly perishable, which may optionally be manufactured in conjunction with an orbital implant if desired, and which provide for a smooth surface covering for orbital implants.

It is another object of the present invention to provide prosthesis comprising an orbital implant with said synthetic coating material having affixed thereto an artificial eye.

It is another object of the present invention to provide integrated orbital implants which are coated so that they may be fully inserted in the orbital socket and permit penetration of fibrovascular tissue.

It is another object of the present invention to provide the orbital implant additionally containing an agent which promotes vascularization.

It is another object of the present invention to provide said orbital implant with synthetic coating material containing, either in association with said implant or in association with said synthetic coating material, an agent which promotes vascularization.

It is another object of the present invention to provide a prosthesis comprising an orbital implant with synthetic coating material with an artificial eye affixed thereto, containing, either in association with the orbital implant or in association with the synthetic coating material, an agent which promotes vascularization.

Set forth below are the preferred embodiments. These embodiments are illustrative, and are not intended to be limitations to the appended claims.

Orbital Implants

The preferred implant is an integrated orbital implant. As set forth above, the term "integrated" herein denotes those implants into which the recipient's own tissue will penetrate as the socket surrounding the implant heals. Other implants which are not "integrated" such as certain acrylic or silicone implants, may also be used, and one skilled in the art will recognize the extent to which the coating or vascularization compositions and methods described herein are applicable thereto.

Low density, porous hydroxyapatite of the kind obtained from coral or by synthetic means is the more preferred material for the integrated orbital implant. Implants made of this materials are available from Integrated Orbital Implants, Inc., San Diego, California. The less preferred material is granular high density hydroxyapatite, such as that used as bone grafting material. For low density hydroxyapatite, a sphere is machined to an appropriate size to be used as an implant from a larger block of porous hydroxyapatite. Hydroxyapatite implants are sterilized, preferably by autoclaving, prior to the use of the implant in the surgical procedure.

As noted above, orbital implants may be used in eviscerations, where the contents of the eyeball is removed, and replaced with the orbital implant; the coating or wrapping is provided by the patient's own scleral sac which is sewn closed around the implant. For enucleation, where the entire eyeball is removed after severing it from the eye muscles and the optic nerve and replaced with an implant; the implant may be placed inside coating material which may be also be sutured closed if appropriate, or the implant may be dipped in coating material if that is appropriate. In addition, the present compositions and procedures are useful for replacing original orbital implants with a second orbital implant. This "secondary" replacement is particularly important as some patients may desire to replace their original non-integrated implant with an integrated orbital implant so as to create a more natural movement, and natural position. This replacement with an additional implant may also be required if the previous implant has migrated, has become exposed, or has been extruded. For secondary implant replacement, the implant, if desired, is placed inside coating material which may be closed via suturing (if appropriate) or the implant may be dipped in coating material prior to use. In the above surgical procedures, the implant or, if coated, the coated implant may then be sutured to the patient's extraocular muscles of the orbit.

Generally, after implantation of an integrated orbital implant, the socket is allowed to heal for approximately six months. During the healing process, fibrovascular tissue penetrates the porous structure of the sphere as the coating material is gradually absorbed or penetrated. After sufficient in-growth, the implant can be drilled or otherwise modified to permit the artificial eye to be coupled to it. U.S. Patent No. 4,976,731, sets forth two embodiments for connecting the orbital implants to the artificial eye. In one embodiment, the artificial eye is permanently fitted with a peg which then fits into a hole drilled into the implant, thus coupling the implant with the artificial eye. In another embodiment, a protruding peg is placed into a drilled hole of the implant and the artificial eye is recessed to receive the peg and thus be coupled with the implant via the peg. In either embodiment, the artificial eye and peg can be readily removed to permit cleaning. One skilled in the art will recognize other means to attach the artificial eye to the implant which has integrated.

One additional means of coupling the implant to the artificial eye is the use of magnets. For example, one pole of the magnet (for example, the "+" pole) is incorporated within a hydroxyapatite orbital implant. The opposite pole of the magnet (in this example, the "-" pole) is incorporated within the artificial eye. The attraction between poles causes the artificial eye to be coupled to the implant.

In this way, the resulting integrated implants are very satisfactory from the patient's point of view. The implant resists extrusion from the orbit. Instead, it becomes an integral part of the orbital structure because of the integration of the fibrovascular tissue into the porous material. Being fixed to the eye muscle, the implant is capable of tracking with the normal eye. When an artificial eye is fixed to the implant to complete the prosthesis, a very satisfactory, natural appearance results.

To date, over 1,000 patients have had this material implanted into the orbit. There have been no chronic infections or extrusions of the implant in patients whose implants have vascularized and have been followed up to 60 months. In those patients who have had the hole drilled into the implant so that it is coupled to the artificial eye, the tracking has been very satisfactory.

Coating Materials

Preferred coating materials for use with integrated orbital implants satisfy two criteria: (1) they will be accepted by the patient, and not cause undue adverse immune response; and (2) ultimately, upon the integration of the orbital implant, the coating material will be absorbed by the patient's body. The term "synthetic" is used herein to denote that the source of material is manufactured in some way, rather than obtained directly from human or other animal sources (such as tissue from the patient himself or eye banks or tissue banks). For example, plaster of Paris or similar calcium-containing materials are appropriate synthetic coating materials. Materials produced in vitro, such as, for example, by recombinant nucleic acid technology or by cell culture techniques, are herein considered synthetic, as are other more traditional means for chemical synthesis. For example, a gene encoding a polymer, such as collagen, or a subunit thereof, may be expressed in cell culture. Also, animal tissue, or substances obtained from animals are considered synthetic in that such materials must be obtained from animal sources and treated for use in humans.

Preferably, the coating material will be a biodegradable, polymeric compound. The term "polymeric" or "polymer" is used here to denote any molecule which is comprised of repeated subunits, including proteinaceous molecules. The term "polymeric compound" refers to a compound which contains at least one polymer. The polymer may be cross-linked, and is preferably elastomeric, as opposed to rigid in structure.

Several biodegradable polymers are currently being investigated for medical applications, such as polyglycolic acid, polylactic acid, polycaprolactone, polydioxanone, polycyanoacrylates, polyorthoesters, poly(gamma-ethyl glutamate), as well as pseudo poly(amino acids). For background on the suitability of these polymers for synthetic coating materials, see Kohn, Medical Designs and Material (March 1991):25-30.

In the case of integrated orbital implants, the coating material should be absorbed by the body because any artificial material left in the body may inhibit epithelialization and may become a site of chronic infections.

Preferably, the outer coating material should be thick enough to make the implant smooth, typically about 0,15875 cm (1/16th of an inch) thick. In addition, the coating material should otherwise allow for the free formation of fibrovascular tissue within the orbital implant. For example, synthetic extracellular matrix proteins, such as the collagens, or other polymers which provide for structural integrity are also suitable as coating or wrapping materials.

One should recognize that the above materials are illustrative of the present synthetic coating materials, and are not intended as an exhaustive list. Other synthetic coating materials will be apparent to one skilled in the art.

The coating material itself should be sterilized, either in conjunction with the orbital implant, or separately and then used to cover the separately-sterilized implant under sterile conditions. Antibiotics may be used as an additional precaution to prevent infection. The choice of sterilization means will depend, in part, on the coating material used. For example, autoclaving may be inappropriate for various polymers, including proteinaceous compounds, as the extreme temperature and pressure may cause a change in the characteristics of the material. One skilled in the art will be able to readily ascertain which sterilization processes are appropriate for which coating materials/orbital implant materials.

Preferably, the coating material is suitable for covering the implant as part of a single manufacturing process. This would benefit the surgeon who would save time by not having to prepare the implant immediately prior to placing the implant into the patient. This would also benefit the patient who would be assured of proper implant characteristics, such as lack of immunogenicity, and ready absorption by the body, for example. This would benefit both the surgeon and the patient because it would decrease the time in which the patient would be under anesthesia. This would also benefit the manufacturer who would be able to place the implant in the synthetic coating as part of the manufacturing process and would be able to store and ship inventory as a single unit. The manufacturer could also ship the coating and the implant as two separate units which could then be assembled by the surgeon.

For example, the synthetic coating material may be in solution or liquid form. The un-coated implant may be dipped directly into the coating material. Or, the synthetic coating material may be in a sheet-like form, and may be wrapped around the implant.

Most preferably, an integrated orbital implant is used with a synthetic coating material, wherein said synthetic coating material causes no undue immune reaction in the patient, is absorbed by the patient's own tissue, and is capable of being vascularized. Particularly, an integrated orbital implant comprised of sterile, low density, porous hydroxyapatite, with sterile coating material including at least one synthetic polymer is the most preferable embodiment. Also, as described below, Plaster of Paris also serves to smooth over orbital implants without the adverse effects described above.

As will be described in further detail below, the coating material may be comprised of synthetic material which is also useful to deliver therapeutic agents, such as vascularization agents. For example, the synthetic material may be a polymer cross-linked in such a way as to provide for time-released drug delivery. The cross-linkages may essentially "trap" aqueous solutions of therapeutic agents, and substantially mediate the release of such agents from the coating materials. Other means of incorporating therapeutic agents into coating materials will be apparent to those skilled in the art.

Therapeutic agents, in addition to agents which promote vascularization, may also include, for example, antibiotic agents, wound healing promoters, blood clotting/blood clot dissolving agents, or mixtures thereof.

Smooth-Coated Hydroxyapatite Implants

One reason for having a smooth surface on the hydroxyapatite orbital implant is primarily to allow it to be placed into the socket without the surface of the hydroxyapatite orbital implant grabbing onto the soft tissues. The surface of the hydroxyapatite orbital implant is covered with many small spicules, and it is very much like Velcro™ when placed in contact with soft tissue--it clings to the tissue very firmly. Therefore, without having a smooth surface, the hydroxyapatite orbital implant cannot easily be placed deep within the orbit.

Coating or wrapping the hydroxyapatite orbital implant in homologous sclera makes the outside surface smooth and allows the implant to go into the orbit in a way similar to a silicone or plastic ball. If it is not wrapped in sclera, the implant can be wrapped with two pieces of thin plastic and then, once the implant is in place, the plastic is withdrawn from the orbit, leaving the hydroxyapatite orbital implant in its proper position in the deep orbit. These materials, however, have drawbacks as described above.

An hydroxyapatite orbital implant with a smooth coating on the outside that is absorbable would make it easy to place the implant deep within the orbital tissues without coating or wrapping it in any other material. For example, plaster of Paris, when used as a coating directly over the hydroxyapatite implant, smooths the surface of the implant and allows for deep orbital implantation. In addition, plaster of Paris is absorbed and allows for penetration of fibrovascular tissue, and avoids immuno-incompatability problems. Also, plaster of Paris is autoclavable, and inexpensive. Other coatings which are calcium-based or otherwise similar to plaster of Paris may also provide these advantages. The description of combining the coatings with other materials, such as therapeutics, may also be applicable herein.

Other coatings which provide for a smooth surface which are capable of being penetrated with fibrovascular tissue and which provides no significant immunoincompatability problems will be apparent to those skilled in the art.

Vascularization Agents

Relatedly, as indicated above, agents which promote vascularization may also be used in conjunction with orbital implants, particularly integrated orbital implants. Generally, the term "promote" with reference to vascularization denotes increasing the rate of blood vessel formation, or increasing the number of blood vessels per unit volume. Typically, the more vascularization is promoted, the sooner the orbital implant is integrated into the patient's orbital socket. Even if integrated orbital implants are not used, improved vascularization in the area surrounding the implant may promote wound healing.

One advantage of having the hydroxyapatite orbital implant impregnated with an agent that causes more rapid vascularization would mean that the patient could be fit with a motility prosthesis more rapidly if the implant is vascularized more rapidly. The motility peg should not be placed within the implant until there is good vascularization of the implant. This takes approximately six months in most patients and even longer in a small number of patients. Once the hydroxyapatite orbital implant is impregnated with fibrovascular tissue, the chances of it migrating are much decreased and the chance of it becoming infected are much decreased.

Other porous implants, in addition to hydroxyapatite orbital implants, are also suitable, and may be vascularized. Preferably, the pores will be interconnected, i.e., the pores will not "dead end." This leads to full vascularization.

Increase in vascularization may be accomplished by vascularization agents, such as growth factors. These growth factors may be applied via the orbital implant itself, for example, by dipping the orbital implant into a solution containing the vascularization agent prior to insertion of the orbital implant. Alternatively, as described above, the vascularization agent may be incorporated into the coating or wrapping material. For example, if a synthetic polymer is used, the polymer may be prepared such that the vascularization promoter is contained within the chains of the polymer molecules. Another alternative is for the implant to be impregnated with a vascularization agent as part of the manufacturing process.

As yet another alternative, exogenous vascularizing agents may be applied as a post-operative therapy to encourage the integration of an integrated orbital implant or promote wound healing. One skilled in the art will envision other means for applying vascularization agents in this context to improve the rate or character of vascularization of the orbital implant.

For example, in the practice of the therapeutic methods, an effective amount of the active compound, including derivatives or salts thereof, or a pharmaceutical composition containing the same, is administered via any of the usual and acceptable methods known in the art, either singly or in combination with another compound or other pharmaceutical agents such as immunosuppressants, antihistamines, corticosteroids, and the like.

These compounds or compositions can thus be administered orally, sutlingually, topically (e.g., on the skin or in the eyes) parenterally (e.g., intramuscularly, intravenously, subcutaneously or intradermally), or by inhalation, and in the form of either solid, liquid, or gaseous dosage including tablets, suspensions, and aerosols, or other forms. The administration can be conducted in single unit dosage form with continuous therapy or in single dose therapy ad libitum. As described above, the compounds also may be delivered via the implant coating or wrapping material, or via the implant itself.

The therapeutic methods are practiced when the relief of symptoms is specifically required or perhaps imminent; in another treatment, the method hereof is effectively practiced as continuous or prophylactic treatment.

In the practice of the therapeutic methods, the particular dosage of pharmaceutical composition to be administered to the subject will depend on a variety of considerations including the nature of the affliction, the severity thereof, the schedule of administration, the age and physical characteristics of the subject and so forth. Proper dosages may be established using clinical approaches familiar to those skilled in the medicinal arts.

Examples of agents which promote vascularization include growth factors, such as epidermal growth factor, fibroblast growth factor, neovascular growth factor, and epithelial growth factor. Also, serum or plasma, preferably from the patient himself to avoid antigenicity or disease transmission problems promotes vascularization. One skilled in the art will be able to ascertain other useful vascularization agents.

These agents which promote vascularization may also be used in conjunction with other agents which produce beneficial effects. For example, as indicated above, immunosuppressant or antibiotic agents may provide beneficial results and prevent undue immune response or insure against undue infection. Certain cell adhesion modulating molecules, such as arginine-glycine-aspartic acid (RGO) containing compounds, or heparin may provide beneficial cell adhesion to the implant and thereby promote integration of integrated implants.

It should also be understood that vascularization agents may be contained in an impure medium or may be contained as a mixture of known ingredients. For example, it has been thought that dipping a hydroxyapatite integrated orbital implant into the patient's own normal human serum or plasma increases the rate and the degree of robustness of the vascularization. One skilled in the art will recognize that there are many vascularizing agents, some of which may also function as wound healing agents, or have other beneficial functions. The above list of vascularization agents is not intended to be complete, and it is not intended to provide limitation to the appended claims.

Brief Description of the Drawings

FIG. 1 is a photographic representation of a porous hydroxyapatite implant wrapped in sclera with windows cut into the sclera for attachment of muscles and for ingrowth of vessels. Similar windows may be cut in suitable synthetic coating or wrapping materials.

FIG. 2 is a technetium[99] bone scan showing good uptake and hence good vascularization of the implant in the right orbit.

FiG. 3 is a photographic representation of an implant showing a peg for direct attachment of an artificial eye.

FIG. 4 is a photographic representation of an implant showing a ball and socket coupling for direct attachment of an artificial eye.

Example

Surgical Technique -- Enucleation

Enucleation is the complete removal of the eyeball after severing it from the eye muscles and the optic nerve. This example describes the surgical technique of enucleation and replacement of the contents in the orbital socket with a hydroxyapatite implant. Coatings and wrappings are discussed.

The enucleation procedure may be done under local or general anesthesia. In either case, 4-5 cc of 2% Lidocaine with epinephrine and Hyaluronidase are given in the retrobulbar space for hemostasis. When a local is being used for the anesthesia, the 2% Xylocaine with epinephrine may be mixed as a 50-50 mix with 0.75% Bupivacaine and Hyaluronidase for a longer duration of effect. A 4-0 silk double-armed suture is used through the upper fornix in a mattress fashion (D.E. Soll Archives of Ophthal., Vol. 87, p. 196 (1972)). This is done to isolate and protect the levator muscle and to identify the superior fornix to keep it from being shortened during closure of anterior Tenon's capsule. In cases of secondary implant, this suture may also be used inferiorly to delineate the desired inferior fornix. A 360-degree peritomy is done preserving as much conjunctiva and Tenon's as possible. The extraocular muscles, including the obliques, are tagged with a double-armed 5-0 Vicryl suture, and the muscles are released from the globe. The neurectomy is done using the surgeon's preference, and the eye is delivered. As in any enucleation procedure, hemostasis should be well-controlled prior to putting an implant in the socket.

At this point, the hole in posterior Tenon's capsule is evaluated where the optic nerve penetrated the capsule. If this opening is small, it is widened by spreading the opening with a blunt hemostat. This will allow the orbital implant to extend into the muscle cone, thus allowing a larger implant to be used. Anterior Tenon's layer can then be closed without tension. This is a variation on the technique first described by Soll (Id.). In the technique described herein, however, posterior Tenon's capsule is not closed anterior to the implant.

The size of the porous hydroxyapatite (PHA) orbital implant to be used is then determined by placing a silicone or acrylic sphere into the orbit. Generally, a 20 mm or 22 mm easily fits into the orbit with anterior Tenon's capsule being pulled together without tension. By evaluating the fit of the silicone or acrylic sphere, the size of the PHA sphere to be used can be chosen.

Experience has shown that the movement of the implant and thus the artificial eye is greater when the implant is coated or wrapped. The use of other wrapping or coating material is herein described. Procedures for using bio-polymers or other materials described herein may be performed prior to the initiation of surgery, if these materials are not rapidly degraded. For example, the entire step of wrapping the implant in sclera is eliminated if the implanted is coated with plaster of Paris. For using bio-polymers, for example, one may essentially coat or wrap the implant in a material comprised of the bio-polymer. This may be done in advance of the surgery, and obviate the need for prolonging the patient's exposure to anesthetics.

In addition, the implant itself may be impregnated with vascularizing agents at this point or previously. For example, the implant may be dipped in the patient's own serum, or other vascularizing agent, prior to surgery. As described above, the vascularizing agent may be in the coating or wrapping material, as well as the implant itself.

For example, an hydroxyapatite orbital implant may be coated in plaster of Paris, autoclaved, and ready for insertion into the recipient. Or, an hydroxyapatite orbital implant may be dipped in a bio-polymer, or dipped in a "cocktail" of biopolymer, therapeutic agent (such as antibiotic), and vascularization agent. Or, an hydroxyapatite orbital implant may be dipped in a vascularization agent, then wrapped in a sheet-like form of a biopolymer, such as a suitable size sheet of collagen which has been prepared in vitro. These examples are illustrative, as a wide variety of combinations nay be selected, which selection may depend on the recipient, the surgeon's own experience, and the materials involved, as well as other factors which will be apparent to one skilled in the art.

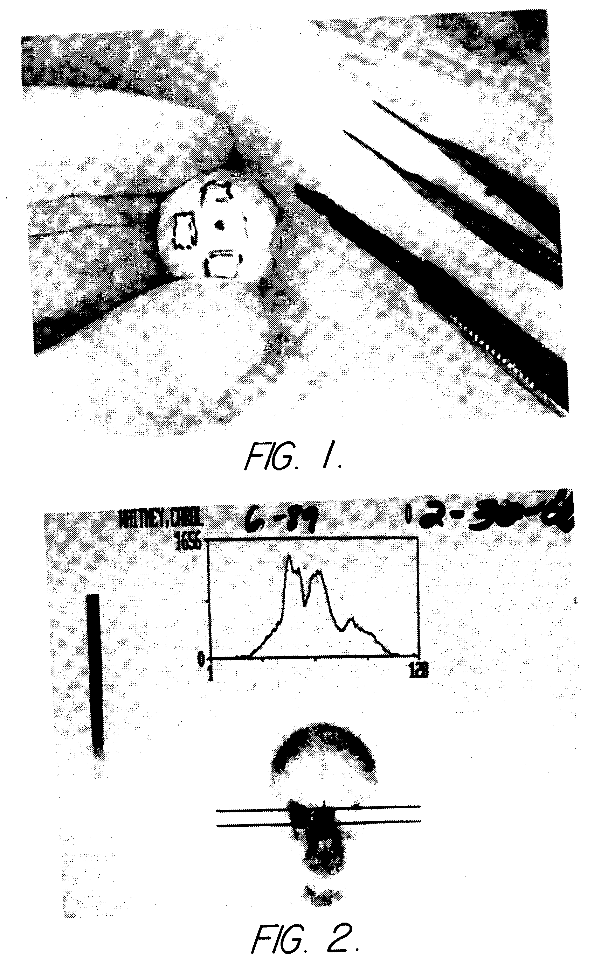

Once the PHA implant has been coated or wrapped in sclera or other material, the anterior pole of the implant is determined and marked with a marking pen. I tend to make an area near the optic nerve the anterior pole. I cut out the cornea and placed it into the socket posteriorly. The area where the rectus muscles are to be attached to the sclera is then determined, and small windows (5 mm x 7 mm) of sclera are cut out (FIG. 1). This area of attachment can be determined by placing the scleral wrapped implant into the orbit in its proper position and marking where the cut ends of the rectus muscles fall on the sclera with normal tension being placed on the muscles. The rectus muscles are then sewn to the sclera by passing the double-armed 5-0 Vicryl through the anterior lip of the scleral window. Tying the suture down snugly pulls the muscle into the window and into contact with the PHA sphere. The obliques are attached at their appropriate positions on the sclera, but windows are not generally cut out for their attachment.

Cutting out the windows in the sclera for the attachment of the muscles and cutting the cornea out and placing it posteriorly, allows more rapid vascularization of the PHA implant. Blood vessels can more rapidly go into the PHA material without having to first penetrate the sclera.

The double-armed sutures anchoring the rectus muscles to the sclera may then be passed through anterior Tenon's and conjunctiva into their respective fornices and tied externally on the conjunctiva. This tends to deepen the fornix. Care should be taken to place these sutures in such a fashion that they do not keep anterior Tenon's capsule and conjunctiva from closing without tension. The anterior Tenon's layer is then closed with interrupted 5-0 Vicryl suture, and the conjunctiva is closed with a running 5-0 Vicryl suture. Antibiotic ointment is applied to the socket and an acrylic conformer is placed in the socket. Care is taken to be sure the conformer does not put undue pressure on the closure of conjunctiva and Tenon's capsule. One or two inter-marginal temporary tarsorrhaphy sutures of 5-0 Vicryl can be used if more than normal edema is anticipated. These intermarginal sutures are routinely used with eviscerations. At the end of the procedure, 4 cc of 0.75% Bupivacaine are injected into the muscle cone for postoperative pain control.

I have used a firm pressure dressing over the orbit and leave it in place for 4 - 5 days. I have the patient on an oral antibiotic for ten days. A four-day course of oral steroids may be used to decrease the swelling of the orbit and therefore decrease the patient's discomfort. After the dressing is removed, the socket is treated with topical antibiotics, and the socket is usually ready for a prosthetic fitting in six weeks.

The PHA implant may be placed in the enucleated socket without being wrapped in sclera or other permanent wrapping. Smooth material may be used to drape the rough implant for insertion. Once inserted, the smooth material may be removed. Holes can be cored through the implant material with a 19-gauge hypodermic needle and a 4-0 Vicryl suture passed through these holes with a Keith needle. The extraocular muscles are then tied to the 4-0 Vicryl suture material that has been passed through the implant. When sclera is not used, I use two pieces of plastic draping material slightly overlapped on each other. The PHA sphere is placed in the center of this overlapped plastic, and the edges of the plastic are drawn up around the implant. The implant then being completely wrapped in this plastic material can be placed into the deep portion of the orbit. The index finger holds the orbital implant in place, and the two pieces of plastic are gently pulled from underneath the implant leaving the implant in its proper position. Tenon' s capsule and conjunctiva are then closed over the implant in a normal fashion.

Since the material has a rough surface and has many small spicule-type projections, when not wrapping the material in sclera or otherwise providing for a smooth surface, extra care should be taken in closing the Tenon's and conjunctiva because the sharp edges of the material may cause the anterior closure to open. If there is any question as to the integrity of the anterior closure, I recommend that a small cap of sclera or fascia be placed between the anterior surface of the implant and the Tenon's closure. This gives another barrier of protection and prevents early exposure. Until this implant becomes vascularized, it should be treated as any other implant, and early exposure should be avoided.

Surgical Technique - Evisceration

The porous hydroxyapatite implant is well-suited to be use with an evisceration. The implant is placed within the patient's own sclera. The implant to be placed within the patient's own sclera may be coated or wrapped prior to insertion in the scleral cavity.

Evisceration, the removal of the intraocular contents of the eye, can be done with the cornea left intact, or with the cornea removed. The procedure can be done under a local or general anesthesia. After the appropriate anesthesia has been given, a lid speculum is place between the lids and a 360 degree peritomy of the conjunctiva is done at the limbus. Dissection between the sclera and Tenon' s capsule is done in the quadrants between the rectus muscles. If the cornea is to be removed, it is removed at this time and a dissection carried out between the choroid and the sclera to remove the intraocular contents. The intraocular contents may be saved as a surgical specimen for examination by pathologists.

If the cornea is left intact, an incision in the sclera approximately 5 to 6 mm. posterior to the limbus just in front of the insertion of the superior rectus muscle is carried out and extended for 180 degrees. The intraocular contents are removed with an evisceration spoon with the dissection being carried out between the sclera and choroid. This specimen is also sent to pathology.

The inside layer of the sclera is scrubbed well to remove any further residue pigment. Also, if the cornea is left intact, the epithelium and the endothelium are removed. The inside of the sclera is then treated with a cotton tip applicator soaked in absolute alcohol to denature any remaining pigmented cells. The inside of the sclera is well irrigated with normal saline solution. Hemostasis is maintained with a cautery unit.

Relaxing incisions in the sclera can be made in between the rectus muscles to allow a larger implant to be placed within the scleral cavity. Also, the sclera can be opened posteriorly to allow a larger implant to be placed within the scleral cavity and also, by opening the sclera in the posterior aspect, this allows more rapid vascularization of the hydroxyapatite implant. The scleral cavity is sized with a silicone or plastic sphere to determine the size of the hydroxyapatite implant. Once the size is determined, the hydroxyapatite implant is placed on the sterile table and soaked in antibiotic solution and saline.

If the implant is not coated, the hydroxyapatite implant is then wrapped in two pieces of thin draping plastic. This is done by placing two 15,24 x 15,24 cm (6x6 inch) pieces of plastic so that the edges overlap by 0,635 cm (1/4 inch). The hydroxyapatite implant which has been chosen is placed in the central portion of this overlapped area, and the plastic is drawn up around the hydroxyapatite implant. This produces a smooth coating for the outside of the implant.

If the implant is already coated with another material, as described above, this would not be necessary. Also, as indicated above, the implant or coating may be treated with vascularization agents or other therapeutic agents. As described above, the implant may be coated or wrapped in synthetic material, such as plaster of Paris, or biopolymers, and vascularizing agents may be used, for example, in conjunction with antibiotics. This prepared implant is then inserted into the sclera.

The implant is then placed within the scleral cavity and if the plastic has been used, the plastic is pulled out from under the implant while the implant is being held in the scleral sac with the index finger. The sclera anteriorly is then closed with interrupted 5-0 Vicryl sutures. Tenon's capsule is then closed over the sclera and/or cornea with interrupted 5-0 Vicryl sutures and then the conjunctiva is closed with a running 5-0 Vicryl suture. A temporary conformer is placed within the lids after antibiotic ointment has been applied, and the lids are temporarily closed with two temporary tarsorrhaphy sutures of 5-0 Vicryl. A firm pressure dressing is applied.

Surgical Technique - Secondary Orbital Implant

The porous hydroxyapatite orbital implant can be used as a secondary implant in patients who have (1) no orbital implant, (2) a migrated orbital implant, (3) an implant with inadequate volume, (4) an extruding orbital implant, or (5) in patients who desire to have more motility of the artificial eye. This procedure can be done under local or general anesthesia.

Surgical Technique

After adequate anesthesia has been given, a double-armed silk suture is placed in the upper and lower fornix to identify the fornices. A lid speculum is placed between the lids. An incision across the posterior aspect of the orbit is made in the horizontal dimension. A conjunctival flap is dissected into the fornix above and below. The orbital implant and its pseudocapsule are removed, if they are present, and a pocket for the new orbital implant is fashioned. The orbital implant can be placed into this pocket, wrapped in sclera or unwrapped and, if the implant surface is smooth (such as if it is pre-coated), no wrapping is necessary. As with the surgical techniques above, the implant may be coated with a variety of materials, and may contain vascularization agents or other therapeutic agents. It should be noted that prior to implanting the hydroxyapatite implant, in this or other surgical procedures, it should be soaked in saline with antibiotic solution.

After placing the implant into the socket, the anterior soft tissues are closed with interrupted 5-0 Vicryl, and the conjunctiva is closed with a running 5-0 Vicryl. If the muscles are to be attached to the implant, after the patient's implant has been removed along with its pseudocapsule, exploration of the orbit is then done in an attempt to locate and isolate the extraocular muscles. Once these have been identified, the hydroxyapatite is placed into the orbit, which may be treated with coatings or vascularizing agents or other agents as described above.

The extraocular muscles are then sutured to the implant material or to the surrounding wrapping, such as if the implant is wrapped in sclera or wrapped in a synthetic coating which is in sheet form. After suturing the muscles to the implant, the anterior soft tissues are closed with interrupted 5-0 Vicryl and the conjunctiva is run with 5-0 Vicryl. A temporary conformer is placed between the lids after antibiotic ointment has been applied, and temporary tarsorrhaphy sutures of 5-0 Vicryl are applied. A firm pressure dressing is then applied.

Drilling the Hole:

The patient that has received a porous hydroxyapatite (PHA) orbital implant is treated in the same way as any other an ophthalmic patient postoperatively. The socket is usually ready for a custom-fit prosthesis in six weeks. There may be slightly more swelling in the early post-operative period because of the movement of the implant in the socket.

The time it takes for the integrated orbital implant to be vascularized is variable. See, D.E. Soll, Advances in Ophthalmic, Plastic and Reconstructive Surgery, Vol. 2, p. 1322, St. Louis, C.V. Mosby Co., 1987. The integrated implant vascularizes more quickly if not wrapped. Having openings cut in the covering of sclera or other appropriate coating or wrapping material, increases the rate of vascularization. If left completely wrapped in sclera, the implant is generally vascularized in about six months. It is likely that other coating or wrapping material, such as synthetic collagen, will require a similar time frame for vascularization in the absence of vascularization agents. And, as indicated above, vascularization agents can be used to improve the time frame for vascularization.

At about six months postoperatively, a technetium[99] bone scan is done to assess the vascularity of the implant (FIG. 2). If the implant is well vascularized, it is ready to be drilled and made into a direct motility implant. One skilled in the art will be able to ascertain the degree of vascularization.

The drilling of the hole for the motility prosthesis is done under retrobulbar (actually retroimplant) local anesthesia. The area on the surface of the conjunctiva to be drilled is marked with a marking pen. This location is most easily determined by having the ocularist make a template of the patient's artificial eye. In the area of the pupil, the template has a through-and-through hole. By placing the template in the socket, the surface of the conjunctiva can be marked through this hole. This indicates the correct location for drilling.

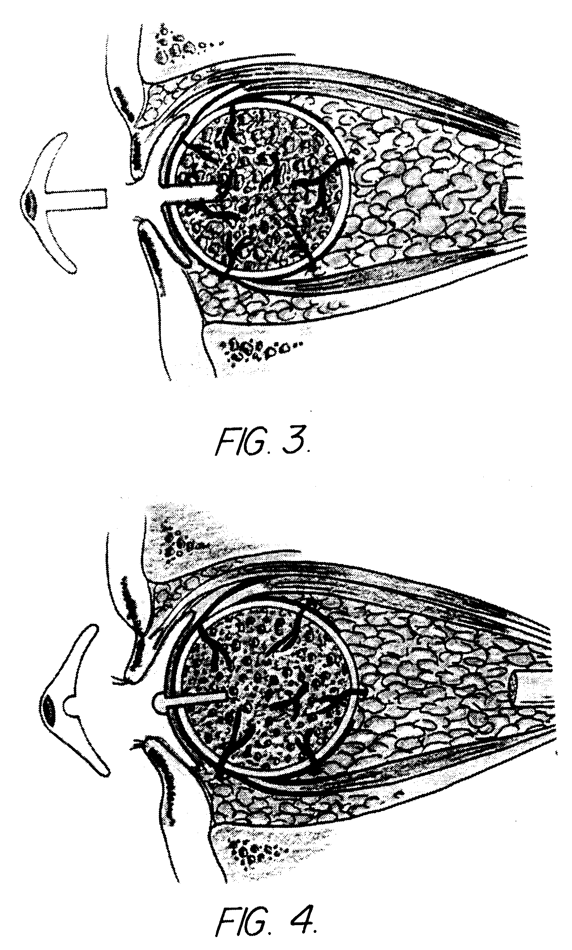

Once the area for drilling has been determined, the eye is prepped and draped in the usual fashion. A lid speculum is placed between the lids, and the area to be drilled is cauterized with a hand-held cautery. The conjunctiva and subconjunctiva tissues are grasped with heavy-toothed forceps to stabilize the implant. A hole 3 mm in diameter and 10 - 13 mm in depth is drilled. It is best to use a drill bit with the cutting portion limited only to the end of the bit and not extending up the sides of the bit. This avoids the conjunctiva from being gathered up onto the drill bit. A flat-headed peg 2.5 mm in diameter and 10 mm long is placed in the drilled hole. If the fit is good and the peg seats well against the conjunctiva, the peg is removed, the hole is irrigated, the peg covered with antibiotic ointment and replaced in the hole. The patient's artificial eye is replaced over this peg, and the eye is patched for 24 hours. In three to four weeks, the patient is sent to an ocularist to have this peg fit to the posterior surface of the artificial eye (FIG. 3). The most common way the implant is coupled to the artificial eye is to have the flat-headed peg replaced with a peg 13 mm long that has a ball on one end. The ball portion of the peg sits above the surface of the conjunctiva. The ocularist then drills a small hemispherical indentation out of the back of the patient's artificial eye, and the ball of the peg fits into this socket. By this ball-and-socket coupling, the movement of the implant is transferred to the artificial eye (FIG. 4). Alternatively, the flat-headed peg can be attached directly to the posterior surface of the artificial eye.

It cannot be emphasized too strongly that the closure of anterior Tenon's and conjunctiva is especially important if the PHA implant is not wrapped in sclera or some other material making a smooth surface. With the amount of movement that the implant will have by being attached to the extraocular muscles, without good closure the tissue may be broken down by the rough edges of the implant material.

Once the implant has been integrated with the fibrovascular tissues of the orbit, exposure of the implant is not of a concern because it will support fibrovascular and epithelial growth on its surface.

Size of the Orbital Implant:

The purpose of an orbital implant is to prevent retraction of orbital tissues, to replace the volume lost by the removal of the eye, to help the prosthesis fit more comfortably and more accurately, and to produce movement of the prosthesis. Replacing the volume of the orbit will have a major effect on the final appearance of the artificial eye.

The volume of the artificial eye plus the volume of the orbital implant should be equal to the volume of the eye that was removed. If the eye is assumed to be a sphere with a diameter of 24 mm, the volume of that sphere is 7.2 cc ( $v=4 \pi R/3$ ). The average artificial eye has a volume of 2.5 cc. Therefore, the volume of the orbital implant should be the difference between the volume of the eye removed (7.2 cc) and the volume of the artificial eye (2.5 cc). This calculation results in an implant that should supply a volume of 4.7 cc (7.2 cc - 2.5 cc). An implant that supplies this volume would have a diameter of 21 mm. Table 1 shows the volume of various implant sizes and the approximate implant sizes to use with eyes of different diameters.

In the past, ophthalmologists have been taught that the largest implant that should be used during an enucleation is 18 mm. It was thought that an implant larger than 18 mm is more likely to be closed with tension on the Tenon's and conjunctival suture line, and therefore, the wound would more likely break down and the implant extrude. The recommended sizes of orbital implants therefore were usually 16 mm or 18 mm. With the use of an 18 mm sphere, a volume deficit of 1.4 cc would occur. The use of a 16 mm sphere will produce a volume deficit of 2.3 cc. This volume deficit of 1.4 cc - 2.3 cc corresponds very well clinically to the volumes often needed to correct enophthalmos with subperiosteal orbital volume augmentation.

I believe the reason that clinicians found the rate of extrusion higher with implants larger than 18 mm was the fact that the implant was placed primarily within Tenon's capsule. With a small opening in posterior Tenon's and the implant placed within the confines of Tenon's capsule, an implant larger than an 18 mm did put undue tension on the anterior Tenon's closure.

Soll made a great contribution in surgical technique with his recommendation of placing the implant within the muscle cone posterior to the deep layer of Tenon's capsule (D.B. Soll, Archives of Ophthal., Vol. 87, p. 196 (1972)). This allowed a larger implant to be placed in the orbit and still have Tenon's capsule closed without tension. Generally, in an adult a 20 mm or 22 mm sphere can easily be placed in the muscle cone and Tenon's be closed without tension.

A large implant (i.e. 22 mm) will be needed if prior to the enucleation a patient has had several surgical procedures or for any other reason may have retraction or fibrosis of the orbital soft tissue or fat atrophy. The wrapping of the implant in eyebank sclera adds about 1.5 mm to the diameter of the implant. Therefore, a 20 mm sphere wrapped in eyebank sclera gives a volume of a sphere with a diameter of 21.5 mm. For sclera-wrapped implants, I prefer to use a 20 or 22 mm PHA implant wrapped in sclera in almost all adult cases with normal-sized globes. Similar techniques can be used for measuring the volume and diameter of implants coated or wrapped in other material.

There can be problems with placing an orbital implant that was too large into the orbit even if extrusion is not a consideration. The problem is that the ocularist may not be able to fit an artificial eye with enough anterior - posterior thickness to create a realistic anterior chamber depth. Also, there may not be enough thickness to allow the ocularist to later drill the posterior surface of the prosthesis to create the socket for the ball-and-socket motility peg. When the prosthesis must be made thin to prevent a proptotic appearance, the anterior chamber depth is shallow and the iris diaphragm appears to be bowed forward. This gives less than a satisfactory result. This is one of the primary problems with the appearance of a scleral shell.

If this were to occur with a PHA spherical implant, the problem would be solved by making an incision over the implant and exposing the implant material. A small drill can then be used to bur away the anterior portion of the implant material and its investing soft tissue. The soft tissue anterior to the implant can then be closed and the volume of the implant reduced. Also, vascularizing materials can be added at this point.

The examples set forth above are illustrative, and are not to be construed as limitations of the appended claims. One skilled in the art will recognize other embodiments of the present invention.

Table 1

| Determination of Orbital Implant Size | | | | |
|---|---|---|---|---|
| (A) EYE SIZE | (B) EYE VOLUME | (C) PROSTHESIS VOLUME | (D) VOLUME TO BE REPLACED | (E) IDEAL IMPLANT SIZE |
| 22 mm | 5.6 cc | 2.5 cc | 3.1 cc | 18 mm |
| 23 mm | 6.4 cc | 2.5 cc | 3.9 cc | 19-20 mm |
| 24 mm | 7.2 cc | 2.5 cc | 4.7 cc | 21 mm |
| 25 mm | 8.2 cc | 2.5 cc | 5.7 cc | 22 mm |
| 26 mm | 9.2 cc | 2.5 cc | 6.7 cc | 23 mm |

**Claims**

1. An orbital implant having a synthetic coating characterized in that said synthetic coating is absorbable by the recipient.

2. The article of Claim 1, wherein the orbital implant is a porous implant.

3. The article of Claims 1 or 2, wherein the orbital implant consists of hydroxyapatite and the hydroxyapatite is porous low density hydroxyapatite or granular high density hydroxyapatite.

4. The article of Claim 3, wherein the hydroxyapatite is obtainable from coral or is synthetic.

5. The article of Claim 1, wherein the orbital implant is a silicone or acrylic implant.

6. The article of any of Claims 1-5, wherein said synthetic coating does not cause an adverse immune response by a recipient.

7. The article of any of Claims 1-6, wherein said synthetic coating is a calcium containing compound.

8. The article of Claim 7, wherein said synthetic material is plaster of paris (2 $CaSO_4$, $H_2O$ [hemihydrate]).

9. The article of any of Claims 1-8, wherein said synthetic coating is prepared by an in vitro technique.

10. The article of Claim 9, wherein said in vitro technique is selected from the group consisting of cell culture, recombinant nucleic acid technology, and a combination thereof.

11. The article of any of Claims 1-6, wherein said synthetic coating contains a polymer.

12. The article of Claim 11, wherein said polymer is selected from the group consisting of polyglycolic acid, polylactic acid, polycaprolactone, polydioxanone, polycyanoacrylate, polyorthoester, poly(gamma-ethyl glutamate), or pseudo poly(amino acids).

13. The article of any of Claims 1-12, wherein said coating includes an extracellular matrix protein.

14. The article of Claim 13, wherein said extracellular matrix protein is selected from the group consisting of collagen, fibrinogen, fibronectin, and vitronectin.

**15.** The article of any of Claims 1-14, wherein said synthetic coating is coated onto the orbital implant by dipping.

**16.** The article of any of Claims 1-14, wherein said synthetic coating is wrapped around the orbital implant.

**17.** The article of Claim 1-16, wherein said synthetic coating comprises a means for allowing an improved vascularization of the implant.

**18.** The article of Claim 17, wherein said means for allowing an improved vascularization of the implant comprise windows through said coating.

**19.** The article of any of Claims 1-18, wherein said synthetic coating material contains a therapeutic agent for delivery.

**20.** The article of Claim 19, wherein said synthetic material is polymer cross-linked in such way as to provide for time-released delivery of the therapeutic agent.

**21.** The article of Claim 19 or 20, wherein said therapeutic agent comprises a vascularization promoting agent, an antibiotic agent, an immunosuppressant, a wound-healing promoter, a blood clot dissolving agent, a blood-clotting agent, a cell adhesion modulating molecule or a combination thereof.

**22.** The article of Claim 21, wherein said vascularization agent is an exogenous vascularizing agent.

**23.** The article of Claim 21, wherein said vascularization agent is a growth factor selected from the group consisting of epidermal growth factor, fibroblast growth factor, neovascular growth factor, or epithelial growth factor, serum or plasma.

**24.** The article of Claim 23, wherein said serum or plasma is autologous.

**25.** The article of Claim 21, wherein said cell adhesion modulating molecule is an arginine-glycine-aspartic acid (RGO) containing compound or heparin.

**26.** The article of any of Claims 1-25 further comprising:

an artificial eye capable of being placed in the orbital cavity along with the orbital implant.

**27.** The article of Claim 26 further comprising:

a means for coupling the artificial eye to the orbital implant.

**28.** The article of Claim 27, wherein said means for coupling the artificial eye to the orbital implant comprises a first magnetic pole in the orbital implant and a second magnetic pole in the artificial eye, said first magnetic pole and said second magnetic pole being oppositely poled.

**29.** The article of any of Claims 26-28, wherein the implant comprises a drilled hole.

**30.** The article of Claim 29 further comprising a peg coupled to the artificial eye and being capable of being placed within said hole.

**31.** The article of Claim 30, wherein said peg is locatable within said hole.

**32.** The article of Claim 33, wherein said peg is a protruding peg

**33.** The article of Claims 1-16, wherein said orbital implant comprises a therapeutic agent.

**34.** The article of claim 33, wherein said therapeutic agent is selected from the group of an antibiotic agent, an immunosuppressant and a cell adhesion modulating molecule.

35. The article of Claim 34, wherein said cell adhesion modulating molecule is an arginine-glycine-aspartic acid (RGO) containing compound or heparin.

36. A method for preparing the article of any of Claims 1-35, wherein the article is manufactured by dipping the uncoated orbital implant into the coating material being present in liquid or solution form, or by wrapping the coating material in a sheet-like form around the uncoated orbital implant.

**Patentansprüche**

1. Ein Augenhöhlenimplantat mit einer synthetischen Beschichtung, dadurch gekennzeichnet, dass die synthetische Beschichtung durch den Empfänger absorbierbar ist.

2. Der Gegenstand nach Anspruch 1, wobei das Augenhöhlenimplantat ein poröses Implantat ist.

3. Der Gegenstand nach den Ansprüchen 1 oder 2, wobei das Augenhöhlenimplantat aus Hydroxylapatit besteht und das Hydroxylapatit poröses Hydroxylapatit niedriger Dichte oder granuläres Hydroxylapatit hoher Dichte ist.

4. Der Gegenstand nach Anspruch 3, wobei das Hydroxylapatit aus Korallen erhältlich ist oder synthetisch ist.

5. Der Gegenstand nach Anspruch 1, wobei das Augenhöhlenimplantat ein Silikon- oder ein Acrylimplantat ist.

6. Der Gegenstand nach irgendeinem der Ansprüche 1-5, wobei die synthetische Beschichtung keine nachteilige Immunantwort durch den Empfänger verursacht.

7. Der Gegenstand nach irgendeinem der Ansprüche 1-6, wobei die synthetische Beschichtung eine Kalzium-enthaltende Verbindung ist.

8. Der Gegenstand nach Anspruch 7, wobei das synthetische Material Gips (2 CaSO$_4$, H$_2$O [Halbhydrat]) ist.

9. Der Gegenstand nach irgendeinem der Ansprüche 1-8, wobei die synthetische Beschichtung durch eine *in vitro* Technik zubereitet wird.

10. Der Gegenstand nach Anspruch 9, wobei die *in vitro* Technik aus der Gruppe, die aus Zellkultur, rekombinanter Nukleinsäuretechnologie und einer Kombination dessen besteht, ausgewählt wird.

11. Der Gegenstand nach irgendeinem der Ansprüche 1-6, wobei die synthetische Beschichtung ein Polymer enthält.

12. Der Gegenstand nach Anspruch 11, wobei das Polymer aus der Gruppe, die aus Polyglykolsäure, Polymilchsäure, Polycaprolacton, Polydioxanon, Polycyanoacrylat, Polyorthoester, Poly(Gamma-Ethylglutamat) oder Pseudo-Polyaminosäuren besteht, ausgewählt wird.

13. Der Gegenstand nach irgendeinem der Ansprüche 1-12, wobei die Beschichtung ein extrazelluläres Matrixprotein einschließt.

14. Der Gegenstand nach Anspruch 13, wobei das extrazelluläre Matrixprotein aus der Gruppe, die aus Kollagen, Fibrinogen, Fibronectin und Vitronectin besteht, ausgewählt wird.

15. Der Gegenstand nach irgendeinem der Ansprüche 1-14, wobei die synthetische Beschichtung auf das Augenhöhlenimplantat durch Eintauchen aufgetragen wird.

16. Der Gegenstand nach irgendeinem der Ansprüche 1-14, wobei die synthetische Beschichtung um das Augenhöhlenimplantat gewickelt ist.

17. Der Gegenstand nach den Ansprüchen 1-16, wobei die synthetische Beschichtung Mittel umfasst, die eine verbesserte Vaskularisation des Implantates erlauben.

18. Der Gegenstand nach Anspruch 17, wobei die Mittel für eine verbesserte Vaskularisation des Implantates Öffnungen durch die Beschichtung umfassen.

19. Der Gegenstand nach irgendeinem der Ansprüche 1-18, wobei das synthetische Beschichtungsmaterial ein therapeutisches Agens zur Abgabe enthält.

20. Der Gegenstand nach Anspruch 19, wobei das synthetische Material ein Polymer ist, das so quervernetzt ist, dass die zeitlich verzögerte Abgabe des therapeutischen Agens ermöglicht wird.

21. Der Gegenstand nach Anspruch 19 oder 20, wobei das therapeutische Agens ein die Vaskularisation förderndes Agens, ein antibiotisches Agens, ein Immunosuppressivum, ein die Wundheilung förderndes Agens, ein Blutgerinnsel auflösendes Agens, ein Blutgerinnungsagens, ein die Zelladhäsion steuerndes Molekül oder eine Kombination davon umfasst.

22. Der Gegenstand nach Anspruch 21, wobei das Vaskularisationsagens ein exogen vaskularisierendes Agens ist.

23. Der Gegenstand nach Anspruch 21, wobei das Vaskularisationsagens ein Serum, Plasma oder ein Wachstumsfaktor ist, der aus der Gruppe, die aus epidermalem Wachstumsfaktor, Fibroplasten-Wachstumsfaktor, neovaskulärem Wachstumsfaktor oder Epithel-Wachstumsfaktor besteht, ausgewählt wird.

24. Der Gegenstand nach Anspruch 23, wobei das Serum oder das Plasma autolog ist.

25. Der Gegenstand nach Anspruch 21, wobei das Zelladhäsion-modellierende Molekül eine Arginin-Glycin-Asparaginsäure (RGO) enthaltende Verbindung oder Heparin ist.

26. Der Gegenstand nach einem der Ansprüche 1-25, der ferner ein künstliches Auge umfasst, das zusammen mit dem Augenhöhlenimplantat in die Augenhöhle eingesetzt werden kann.

27. Der Gegenstand nach Anspruch 26, der ferner Mittel zum Koppeln des künstlichen Auges an das Augenhöhlenimplantat umfasst.

28. Der Gegenstand nach Anspruch 27, wobei die Mittel zum Koppeln des künstlichen Auges an das Augenhöhlenimplantat einen ersten Magnetpol in dem Augenhöhlenimplantat und einen zweiten Magnetpol in dem künstlichen Auge umfassen und der erste Magnetpol und der zweite Magnetpol entgegengesetzt gepolt sind.

29. Der Gegenstand nach irgendeinem der Ansprüche 6-8, wobei das Implantat eine gebohrte Öffnung umfasst.

30. Der Gegenstand nach Anspruch 29, der ferner einen Stift umfasst, der mit dem künstlichen Auge verbunden und in die Öffnung eingesetzt zu werden kann.

31. Der Gegenstand nach Anspruch 30, wobei der Stift in der Öffnung angebracht werden kann.

32. Der Gegenstand nach Anspruch 31, wobei der Stift ein überstehender Stift ist.

33. Der Gegenstand nach den Ansprüchen 1-16, wobei das Augenhöhlenimplantat ein therapeutisches Agens umfasst.

34. Der Gegenstand nach Anspruch 33, wobei das therapeutische Agens aus der Gruppe, die aus einem antibiotischen Agens, einem Immunosuppressivum und einem Zelladhäsion-modellierenden Molekül besteht, ausgewählt wird.

35. Der Gegenstand nach Anspruch 34, wobei das Zelladhäsion-modellierende Molekül eine Arginin-Glycin-Asparaginsäure (RGO) enthaltende Verbindung oder Heparin ist.

36. Ein Verfahren zur Herstellung des Gegenstandes nach irgendeinem der Ansprüche 1-35, wobei der Gegenstand durch Eintauchen des unbeschichteten Augenhöhlenimplantates in das Beschichtungsmaterial, das in einer flüssigen Form oder als Lösung vorliegt, oder durch Wickeln des Beschichtungsmaterials in einer schichtartigen Form um das unbeschichtete Augenhöhlenimplantat herum hergestellt wird.

**Revendications**

1. Implant orbitaire présentant un revêtement synthétique caractérisé en ce que ledit revêtement synthétique est absorbable par le bénéficiaire.

2. Implant selon la revendication 1 dans lequel l'implant orbitaire est un implant poreux.

3. Implant selon l'une des revendications 1 ou 2 dans lequel l'implant orbitaire consiste en hydroxyapatite et l'hydroxyapatite est une hydroxyapatite poreuse de faible densité ou une hydroxyapatite granulaire de forte densité.

4. Implant selon la revendication 3 dans lequel l'hydroxyapatite peut être obtenue à partir de corail ou est synthétique.

5. Implant selon la revendication 1, dans lequel l'implant orbitaire est en silicone ou en acrylique.

6. Implant selon l'une quelconque des revendications 1 à 5 dans lequel ledit revêtement synthétique ne provoque pas de réponse immunitaire défavorable chez le bénéficiaire.

7. Implant selon l'une quelconque des revendications 1 à 6, dans lequel ledit revêtement synthétique est à base d'un composé contenant du calcium.

8. Implant selon la revendication 7, dans lequel ledit composé synthétique est un plâtre de paris ($2 CaSO_4$, $H_2O$ [hémihydrate]).

9. Implant selon l'une quelconque des revendications 1 à 8 dans lequel ledit revêtement synthétique est préparé par une technique in vitro.

10. Implant selon la revendication 9, dans lequel ladite technique in vitro est choisie dans le groupe constitué d'une culture cellulaire, de la technologie de l'acide nucléique recombinant et d'une combinaison de ces techniques.

11. Implant selon l'une quelconque des revendications 1 à 6, dans lequel ledit revêtement synthétique contient un polymère.

12. Implant selon la revendication 11 dans lequel ledit polymère est choisi dans le groupe constitué du poly(acide glycolique), du poly(acide lactique), de la polycaprolactone, de la polydioxanone, du polycyanoacrylate, du polyorthoester, du poly(gamma-glutamate d'éthyle) ou d'un pseudo poly(amino acide).

13. Implant selon l'une quelconque des revendications 1 à 12 dans lequel ledit revêtement comprend une protéine matricielle extracellulaire.

14. Implant selon la revendication 13 dans lequel ladite protéine matricielle extracellulaire est choisie dans le groupe constitué du collagène, du fibrinogène, de la fibronectine et de la vitronectine.

15. Implant selon l'une quelconque des revendications 1 à 14 dans lequel ledit revêtement synthétique est appliqué sur l'implant orbitaire par immersion.

16. Implant selon l'une quelconque des revendications 1 à 14 dans lequel ledit revêtement synthétique est enveloppé autour de l'implant orbitaire.

17. Implant selon l'une quelconque des revendications 1 à 16 dans lequel ledit revêtement synthétique comprend des moyens permettant une vascularisation améliorée de l'implant.

18. Implant selon la revendication 17 dans lequel lesdits moyens permettent une vascularisation améliorée de l'implant comprennent des fenêtres traversant ledit revêtement.

19. Implant selon l'une quelconque des revendications 1 à 18 dans lequel ledit matériau de revêtement synthétique confient un agent thérapeutique à libérer.

20. Implant selon la revendication 19 dans lequel ledit matériau synthétique est un polymère réticulé, de façon à assurer une libération contrôlée de l'agent thérapeutique.

**21.** Implant selon la revendication 19 ou la revendication 20, dans lequel ledit agent thérapeutique comprend un agent facilitant la vascularisation, un agent antibiotique, un immunosuppresseur, un agent facilitant la guérison de blessures, un agent capable de dissoudre les caillots de sang, un agent coagulant, une molécule capable de moduler l'adhésion cellulaire ou une combinaison de ces agents.

**22.** Implant selon la revendication 21 dans lequel ledit agent pour la vascularisation est un agent exogène pour la vascularisation.

**23.** Implant selon la revendication 21 dans lequel ledit agent pour la vascularisation est un facteur de croissance choisi dans le groupe constitué du facteur de croissance de l'épiderme, du facteur de croissance des fibroblastes, du facteur de croissance néovasculaire ou du facteur de croissance épithélial, où ledit agent pour la vascularisation est un sérum ou un plasma.

**24.** Implant selon la revendication 23 dans lequel ledit sérum ou plasma est autologue.

**25.** Implant selon la revendication 21 dans lequel ladite molécule capable de moduler l'adhésion cellulaire est un composé contenant arginine-glycine-acide aspartique (RGO) ou est de l'héparine.

**26.** Implant selon l'une quelconque des revendications 1 à 25 comprenant en outre un oeil artificiel capable d' être placé dans la cavité orbitaire ensemble avec l'implant orbitaire.

**27.** Implant selon la revendication 26 comprenant en outre des moyens pour le couplage de l'oeil artificiel à l'implant orbitaire.

**28.** Implant selon la revendication 27 dans lequel lesdits moyens pour le couplage de l'oeil artificiel à l'implant orbitaire comprennent un premier pôle magnétique dans l'implant orbitaire et un second pôle magnétique dans l'oeil artificiel, ledit premier pôle magnétique et ledit second pôle magnétique étant polarisés de façon opposée.

**29.** Implant selon l'une quelconque des revendications 26 à 28 dans lequel l'implant comprend un trou foré.

**30.** Implant selon la revendication 29 comprenant en outre une cheville couplée à l'oeil artificiel et étant capable d'être placée au sein dudit trou.

**31.** Implant selon la revendication 30 dans lequel ladite cheville peut être localisée dans ledit trou.

**32.** Implant selon la revendication 31 dans lequel ladite cheville est une cheville protubérante.

**33.** Implant selon l'une quelconque des revendications 1 à 16 dans lequel ledit implant orbitaire comprend un agent thérapeutique.

**34.** Implant selon la revendication 33 dans lequel ledit agent thérapeutique est choisi dans le groupe constitué d'un agent antibiotique, d'un immunosuppresseur et d'une molécule capable de moduler l'adhésion cellulaire.

**35.** Implant selon la revendication 34 dans lequel ladite molécule capable de moduler l'adhésion cellulaire est un composé contenant arginine-glycine-acide aspartique (RGO) ou est de l'héparine.

**36.** Procédé pour la préparation d'un implant selon l'une quelconque des revendications 1 à 35 dans lequel l'implant est fabriqué par immersion de l'implant orbitaire non revêtu dans le matériau de revêtement présent sous forme liquide ou en solution, ou en enveloppant le matériau de revêtement en forme de feuille autour de l'implant orbitaire non revêtu.

FIG. 1.

FIG. 2.

FIG. 3.

FIG. 4.